# EUROPEAN PATENT SPECIFICATION

(11) **EP 1 117 981 B1**
(45) Date of publication and mention of the grant of the patent: **11.04.2007**
(21) Application number: 99934742.0
(22) Date of filing: 06.07.1999
(51) Int. Cl.: G01N 11/02, G01N 33/34

(54) **METHOD AND MEASURING APPARATUS FOR MEASURING FREENESS**
VERFAHREN UND MESSGERÄT ZUR BESTIMMUNG DER ENTWÄSSERUNGSNEIGUNG
PROCEDE ET APPAREIL DE MESURE DE L'EGOUTTAGE

(30) Priority: 07.07.1998 FI 981559
(43) Date of publication of application: 25.07.2001
(73) Proprietor: Metso Automation Oy, 00880 Helsinki (FI)
(72) Inventor: KÄHKÖNEN, Martti, FIN-88600 Sotkamo (FI)
(74) Representative: TBK-Patent
(86) International application number: PCT/FI1999/000598
(87) International publication number: WO 2000/002032

(56) References cited:
- DE-A1- 19 635 318
- GB-A- 2 001 117
- US-A- 5 340 442
- US-A- 5 365 775
- ANONYMOUS: "PULP EXPERT GUIDE BOOK NR 1 - Measurement Methods, VERSION 5.0" October 1996 (1996-10), PULP EXPERT OY , XP002374581 * page 23 - page 26 *

## Description

### FIELD OF THE INVENTION

The invention relates to a method for measuring freeness, in which method a measuring chamber comprising a wire or the like arranged on the bottom surface of the measuring chamber is filled with a suspension to be measured and the suspension is allowed to flow through the wire or the like at a time instant T0 and after the flow starts at the time instant T0, the decrease in the suspension in the measuring chamber is measured as a function of time.

The invention further relates to a measuring apparatus to measure freeness, which measuring apparatus comprises a measuring chamber comprising a top lid and a bottom surface shutter that tightly seal the measuring chamber, an air valve and a wire or the like; in the beginning of the measuring process, the measuring chamber contains the suspension to be measured ; the shutter of the measuring chamber bottom surface is made to be opened; the measuring apparatus is adapted to allow the suspension to flow through the wire or the like at a time instant T0 and comprises a measuring sensor for measuring the drainage of the liquid from the measuring chamber through the wire or the like as a function of time.

### BACKGROUND OF THE INVENTION

To make good quality paper, the properties of paper stock must be precisely measured and controlled. In measuring the freeness of paper stock, the speed with which the paper stock can be separated from water is empirically determined. Freeness depends on several factors, such as fibres, stock processing (for instance mechanical/chemical), the quantity of fines, temperature, consistency, and the measuring apparatus.

One of the most common methods for measuring freeness is CSF (Canadian Standard Freeness). This measuring method is a standard and has been disclosed in detail in publication T 227 om-85, Freeness of pulp, TAPPI, 1985. In the CSF measurement, the freeness of paper stock is measured from a sample with 0.3% consistency and 20°C temperature. If the consistency or temperature of the sample differs from the specified values, the freeness result is adjusted according to predefined table values so that the measurement corresponds to the specified consistency and temperature values. In the beginning of the CSF measurement, exactly one litre of the sample is measured into a measuring tank comprising the walls of the tank, a top lid that closes against the top part of the walls, a wire at the bottom of the tank, a bottom lid that closes against the bottom part of the walls, and an air valve. The bottom lid is opened and the sample is allowed to settle in the tank so that some of the stock descends on the wire at the bottom of the tank. After approximately 5 s from opening the bottom lid, the air valve is opened so that water starts separating from the stock sample through the wire and the stock piled on the wire. The water flows into a funnel comprising a constant flow spout at the bottom of the funnel and a lateral tube in the bottom section of the funnel. A constant volume (24.2 ml) remains in the funnel between the constant flow spout (constant flow 8.83 ml/s) and the lateral tube. When water flows from the measuring tank into the funnel, part of the water flows out through the constant flow spout, a constant volume (24.2 ml) of water collects between the constant flow spout and the lateral tube and finally some of the water flows out through the lateral tube. In measuring freeness, this volume of water that has flown out through the lateral tube is measured in a measuring glass and this volume of water corresponds to freeness. The measuring is usually performed manually. The measuring method is arduous and sensitive to changes in temperature and consistency. The measuring method is also inaccurate with low CSF values.

Another known method for defining freeness is the Schopper-Riegler method disclosed in publication SCAN-C 19:65, Scandinavian pulp, paper and board, Testing committee, approved 1964. According to this standard method, a known quantity of paper stock is first poured on a spreader cone which is opened after a predefined period of time (5 s), the stock is filtered through a wire and a mat of fibre piling on the wire into a funnel with an orifice at the bottom and the side. Water flows out through the bottom orifice at a constant flow rate [1000 ml/(149 s ± 1) ≈ 6.71 ml/s]. A constant volume (7.5 ml - 8.0 ml) remains between the bottom orifice and the side orifice. The volume of water flowing through the side orifice corresponds to freeness measured in SR units so that 0 ml corresponds to 100 SR units, 1000 ml corresponds to 0 SR units and thus one SR unit corresponds to 10 ml. The SR and CSF scales are reversed in relation to each other, i.e. the highest SR value corresponds to the lowest CSF value. This measuring is also usually performed manually. The measuring method is arduous and sensitive to changes in temperature and consistency. The SR measuring method is also inaccurate with extreme values.

Patent publication US 2 602 325, discloses a method for measuring freeness similar to CSF measuring, in which freeness is determined by measuring the time that elapses when a predefined volume of liquid separates from a suspension in a measuring chamber. Alternatively, it is possible to measure the volume of liquid that separates from a suspension in a measuring chamber during a predefined time. This solution provides the disadvantage of being slow and susceptible to human error.

Freeness can also be measured with an automated measuring apparatus which resembles CSF measuring in principle, but uses vacuum. A sampling apparatus of the measuring apparatus takes a sample of paper stock, and water is added to the sample so that its consistency becomes approximately 3%. The temperature of the sample is measured and the sample is allowed to descend and settle on the wire for 5 seconds. After this, the water is drained from the tank through the wire using vacuum. After a certain period of time, the pressure difference caused by the cake piled on the wire is measured. After the pressure difference has been measured, the consistency of the paper stock is measured using the mass of the cake. Freeness can be calculated from the pressure difference. The disadvantage of this measuring method is that pattern making (calibration) is arduous and difficult, because the apparatus must be calibrated separately for each stock type.

Patent publication FI 80 342, also discloses an automated method and apparatus for defining the dry stuff, freeness and wire retention of pulp. The measuring of freeness is based on measuring the liquid level of a suspension in a measuring chamber as a function of time. The drainage rate is formed as a function of the mass of the dry stuff cake. The measuring requires a precise weighing machine so as to avoid errors in the freeness result. A weighing machine also increases the manufacturing costs of the apparatus.

Patent publication FI 51 133, discloses another automated method and apparatus for measuring freeness by directing pressurized water through a layer of stock and determining the freeness resistance by the volume of water that flows through during a certain period of time. The disadvantage of this measuring method, too, is that calibration is arduous and difficult, because the apparatus must be calibrated separately for each stock type. Pressurized measuring differs from standard measuring and the results are thus not comparable.

A method and apparatus for measuring freeness by monitoring the drainage of a pulp suspension through a wire as a function of time is also known from publication "Pulp Expert Guide Book Nr 1 - Measurement Methods, Version 5.0" published by Pulp Expert Oy in 1996. Here no vacuum or weighing is used, but comparability with standard methods is achieved through comparison tables only.

### BRIEF DESCRIPTION OF THE INVENTION

It is an object of the invention to solve the above-mentioned problems by implementing a method and an apparatus implementing the method. This object is achieved by a method described in the introduction and characterized in that in the method, a time instant T1 is searched for, at which the decrease in the suspension substantially corresponds to a previously known flow rate v_{c}; and freeness F is determined as a function of the volume of suspension drained from the measuring chamber by the time instant T1.

The measuring apparatus of the invention is characterized in that it comprises an automatic data processing apparatus to which the sensor is made to feed its measuring data; the automatic data processing apparatus is adapted to search for a time instant T1 at which the drainage of the liquid from the measuring chamber substantially corresponds to a previously known flow rate v_{c}; and the automatic data processing apparatus is adapted to determine freeness F as a function of the volume of suspension drained from the measuring chamber by the time instant T1.

The method and system of the invention provide several advantages. Measuring freeness becomes faster, more accurate and simple. Measuring does not require calibration and the measuring apparatus need not be set to the type of stock used in measuring. Further, repeatability improves, as the human factor involved in the measuring can be minimized. The measuring apparatus of the invention is also inexpensive and quick to take into use.

### BRIEF DESCRIPTION OF THE DRAWINGS

In the following the invention will be described in greater detail in connection with preferred embodiments and with reference to the attached drawings, in which
Figure 1 shows a method of the invention,
Figure 2 shows a measuring apparatus of the invention, and
Figure 3 shows a freeness measuring method of the invention in relation to a standard measuring method.

### DETAILED DESCRIPTION OF THE INVENTION

The solution of the invention is especially suitable for paper industry, but is not restricted to it.

Let us first briefly examine the method of the invention. In the solution of the invention the volume of a liquid in a measuring chamber is monitored as a function of time from a time instant T0. When the derivative of the volume of the liquid in relation to time, which corresponds to the flow rate of the liquid draining from the measuring chamber through a wire, reaches a predefined value v_{c} at a time instant T1, a total volume of liquid Vₜₒₜₐₗ drained from the measuring chamber up till then, i.e. between T1 and T0, is calculated. A volume V_{cf} that would correspondingly have drained during the same time T1 to T0 through a constant flow spout, i.e. V_{cf} = v_{c} * (T1 - T0), is subtracted from the total volume Vₜₒₜₐₗ. In the CSF method, constant flow v_{c} is 8.83 ml/s and in the SR method, v_{c} is 6.71 ml/s, i.e. more specifically 1000 ml/T_{vc}, where T_{vc} is 149 s ± 1 s. Additionally, a constant volume, which corresponds to a threshold value state Vₜₕ between a constant flow spout and a lateral tube of a lower funnel, is subtracted from the total volume. In the CSF method, the threshold value state is Vₜₕ = 24.2 ml and in the SR method, it is Vₜₕ = 7.5 to 8.0 ml. Thus according to the method of the invention, freeness F is a function of the differences V = Vₜₒₜₐₗ - V_{cf} - Vₜₕ, i.e. F = f(V), which corresponds to CSF and SR standard measuring. The CSF result directly and substantially corresponds to the difference result V, i.e. F = V. In calculating freeness F, it is, however, also possible to use some other suitable function based on practical experience. In the SR method, the SR scale increases in a different direction than in the CSF method, which means that freeness F in the SR method is nearly directly F = (1000 ml - V)/10 or some other suitable function based on practical experience. The dependency between the SR and CSF methods is non-linear due to, for instance different wires and air cocks when a suspension is allowed to flow through a wire. In the method of the invention, it is naturally possible to freely select any required values for the constant flow v_{c} and the threshold value state Vₜₕ.

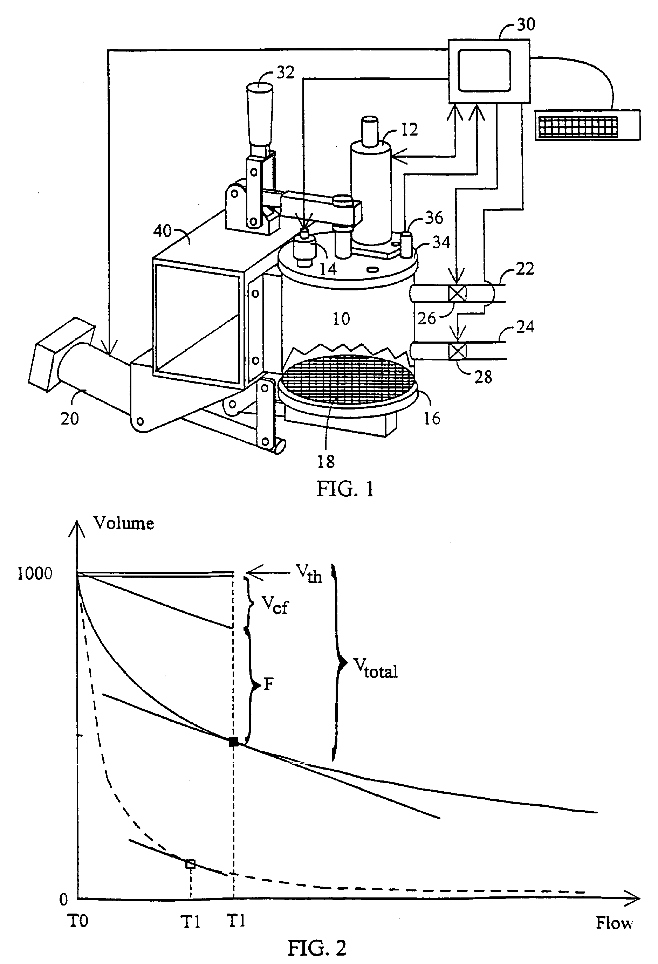

Let us now examine the method of the invention using Figure 1. The measuring apparatus of the invention implementing the method of the invention comprises a measuring chamber 10 comprising a top lid 34 and a bottom lid 16 that tightly seal the measuring chamber 10, and an air valve 14. The measuring chamber is attached to a supporting structure 40. When measuring is started, the measuring chamber 10 is filled with a suspension to be measured. The filling may be performed manually by opening the top lid 34 with a lever 32 and then pouring the suspension into the measuring chamber 10, or automatically through tube 22. Manual filling is not recommended in industrial processes and thus a manual opening mechanism of the top lid is not essential. When the filling is performed through tube 22, an automatic data processing apparatus 30, which is for instance a computer with a microprocessor, opens a valve 26 and the suspension starts to flow into the measuring chamber 10. When the measuring chamber 10 is full, a bottom lid 16 is opened with an opening mechanism 20. After the bottom lid 16 has been opened, the air valve 14 is opened after a predefined delay, usually 5 s, at a time instant T0. Opening the bottom lid 16, measuring the delay and controlling the opening of the air valve 14 are precisely executed by the automatic data processing apparatus 30. The measuring apparatus comprises measuring means 12 for measuring as a function of time the drainage of the liquid from the measuring chamber after the air valve has been opened. The liquid flows through a wire or perforated plate 18 leaving the solid matter in the suspension on the wire or perforated plate 18. The outflow of the liquid is measured with a sensor 12 comprising, for instance an optical or ultrasonic transmitter-receiver pair. The sensor 12 is connected to the automatic data processing apparatus 30. The measuring is performed, for instance so that the optical or acoustic transmitter sends a measuring signal towards the surface of the suspension, from which the signal is reflected to the optical or acoustic receiver. When the location of the transmitter and receiver and the travel time of the signal from the transmitter to the receiver is known, it is possible to specify the level of the surface. The sensor 12 feeds the measuring data of the signal travel time into the automatic data processing apparatus 30 which specifies the flow rate. The automatic data processing apparatus 30 determines freeness F from the collected measuring data according to the method of the invention.

Let us now examine the method of the invention using Figure 2 which shows two curves describing the drainage of the liquid from the measuring chamber 10. In the case of the upper curve, the liquid flows more slowly out of the measuring chamber 10 than in the case of the lower curve (marked with a dash line). The upper curve shows how to read from the curve the total volume Vₜₒₜₐₗ of drained liquid, the volume flowing out at constant flow V_{cf}, freeness F according to the CSF method and the time instant T1, at which the liquid is flowing out from the measuring chamber at a previously known constant flow rate. The lower curve only marks the time instant T1. The slope of the straight line, i.e. the derivative of the curve, shown as the tangent of the curve at the location of the time instant T1 corresponds to the constant flow v_{c}. Typically, the measuring chamber 10 has a capacity of 1000 ml, but the capacity of the measuring chamber 10 is not essential for the solution of the invention. The automatic data processing apparatus 30 searches for, on the basis of the measuring data, a time instant T1, at which the outflow of the liquid from the measuring chamber 10 corresponds substantially to a previously known flow rate v_{c} which is the constant flow 8.83 ml/s (in the SR method, the constant flow is approximately 6.71 ml/s) of the constant flow spout of the lower funnel preferably used in the CSF standard measuring of freeness. In the CSF standard measuring, when 8.83 ml/s (in the SR method, 6.71 ml/s) of liquid or less flows through the wire, liquid no longer flows from the lateral tube of the lower funnel to the measuring vessel measuring freeness, but the liquid flows out through the constant flow spout. To measure freeness, the automatic data processing apparatus 30 determines, in the solution of the invention, the total volume Vₜₒₜₐₗ of liquid drained from the measuring chamber 10 from the time when the air valve was opened at the time instant T0 to the time instant T1. The automatic data processing apparatus 30 also calculates the constant flow volume V_{cf} from the time instant T0 to the time instant T1 so that the constant flow V_{cf} is V_{cf} = v_{c} * (T1 - T0). In the CSF standard measuring, the constant flow volume V_{cf} corresponds to the volume of liquid flown through the constant flow spout. The automatic data processing apparatus 30 calculates freeness F of the suspension by subtracting the constant flow volume V_{cf} and the previously known threshold volume Vₜₕ from the total volume Vₜₒₜₐₗ of liquid drained from the measuring chamber 10. For instance, freeness F of the CSF method corresponds directly and substantially to the difference result V = F = Vₜₒₜₐₗ - V_{cf} - Vₜₕ. The method of the invention is suitable for measuring freeness according to both the CSF standard and the SR standard. When the measuring has been performed, the measuring chamber can be cleaned, for instance with pressurized water through a tube 24 when its shutter 28 is opened. The opening of the shutter 28 is preferably controlled by the data processing apparatus 30 which, in the solution of the invention, also measures the temperature of the suspension with a thermometer 36. The data processing apparatus also preferably measures the consistency of the suspension. The consistency and temperature of the suspension being measured in the measuring chamber is controlled by the data processing apparatus 30. The consistency is preferably 0.3% and the temperature 20°C. When the temperature and the consistency differ from the specified values, the data processing apparatus 30 corrects the freeness result correspondingly according to the CSF standard table. The data processing apparatus 30 is connected or integrated to the data network or control system of the process.

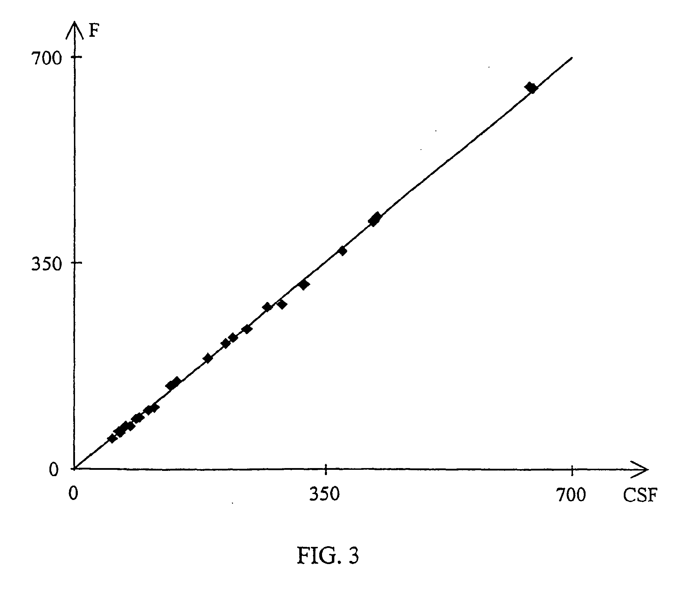

Figure 3 shows the freeness results F measured with a measuring apparatus of the invention on the vertical axis and the freeness results CSF according to the CSF standard on the horizontal axis. Freeness has been measured extensively between 50 and 650. The correlation of the results is almost exactly one, i.e. the solution of the invention produces freeness results very closely corresponding to the CSF standard measurements. In fact, the small differences in the results are caused by errors in the CSF standard measuring. As the CSF standard measuring is performed manually, small errors occur easily, for instance in measuring time (5 s), opening the bottom lid and reading the measuring results, whereas the measuring of the invention is fully automated and performed in exactly the same way every time.

Although the invention is described herein with reference to examples in accordance with the accompanying drawings, it is obvious that the invention is not to be so limited, but the invention may be modified in a variety of ways within the scope of the appended claims.

## Claims

1. A method for measuring freeness, in which method:
- a measuring chamber (10) comprising a wire (18) or the like arranged on the bottom surface of the measuring chamber (10) is filled with a suspension to be measured,
- the suspension is allowed to flow through the wire (18) or the like at a time instant T0,
- after the flow starts at the time instant T0, the decrease in the suspension in the measuring chamber (10) is measured as a function of time; **characterized in that** in the method:
- a time instant T1 is searched for, at which the decrease in the suspension substantially corresponds to a previously known flow rate v_{c}; and
- freeness F is calculated as a function of the volume of suspension drained from the measuring chamber (10) by the time instant T1.

2. A method as claimed in claim 1, **characterized in that** to calculate freeness F as a function of the volume of suspension drained by the time instant T1:
- a total volume Vₜₒₜₐₗ drained from the measuring chamber (10) is determined from the time instant T0 to the time instant T1;
- a constant flow volume V_{cf} is calculated from the time instant T0 to the time instant T1 while the constant flow v_{c} is previously known;
- a difference result V is calculated by subtracting the constant flow volume V_{cf} and a previously known threshold volume Vₜₕ from the total volume Vₜₒₜₐₗ of liquid drained from the measuring chamber (10), and freeness F of the suspension is calculated as a function of the difference result V.

3. A method as claimed in claim 1, **characterized in that** the outflow of the suspension draining from the measuring chamber (10) is measured as a function of time until the flow substantially corresponds to the previously known flow rate v_{c} or is slower than V_{c}.

4. A method as claimed in claim 1, **characterized in that** the temperature of the suspension is measured and the calculated freeness F is adjusted according to the temperature.

5. A method as claimed in claim 4, **characterized in that** temperature adjustment is performed according to a standard table.

6. A method as claimed in claim 1, **characterized in that** the consistency of the suspension is measured and the calculated freeness is adjusted according to the consistency.

7. A method as claimed in claim 6, **characterized in that** consistency adjustment is performed according to a standard table.

8. A method as claimed in claim 1, **characterized in that** the drainage of the liquid from the measuring chamber (10) is measured acoustically by measuring the surface level of the suspension in the measuring chamber (10).

9. A method as claimed in claim 1, **characterized in that** the drainage of the liquid from the measuring chamber (10) is measured optically by measuring the surface level of the suspension in the measuring chamber (10).

10. A method as claimed in claim 1, **characterized in that** in the method all measures and the result calculation are performed automatically using microprocessor control.

11. A method as claimed in claim 2, **characterized in that** the previously known flow rate is v_{c} = 8.83 ml/s and the previously known threshold volume is Vₜₕ = 24.2 ml and freeness F according to the CSF method is substantially F = V or a corresponding value.

12. A method as claimed in claim 2, **characterized in that** the previously known flow rate is v_{c} = 1000 ml/(149 s ± 1 s) and the previously known threshold volume is Vₜₕ = 7.5 - 8.0 ml and freeness F according to the SR method is substantially F = (1000 ml - V)/10 or a corresponding value.

13. A method as claimed in claim 1, **characterized in that** the suspension to be measured remains in the measuring chamber (10) for a predefined time before it is let flow through the wire (18) or the like.

14. A method as claimed in claim 13, **characterized in that** the predefined time is 5 s.

15. A measuring apparatus for measuring freeness, which measuring apparatus comprises a measuring chamber (10) comprising a top lid (34) and a bottom surface shutter (16) that tightly seal the measuring chamber (10), an air valve (14) and a wire (18) or the like; in the beginning of the measuring process, the measuring chamber (10) contains the suspension to be measured; the shutter (16) of the measuring chamber (10) bottom surface is made to be opened; the measuring apparatus is arranged to allow the suspension to flow through the wire (18) or the like at a time instant T0, wherein
- the measuring apparatus comprises a measuring sensor (12) for measuring the drainage of the liquid from the measuring chamber (10) through the wire (18) or the like as a function of time; and
- the measuring apparatus comprises an automatic data processing apparatus (30) to which the sensor is made to feed its measuring data; **characterized in that**:
- the automatic data processing apparatus (30) is adapted to search for a time instant T1 at which the drainage of the liquid from the measuring chamber (10) substantially corresponds to a previously known flow rate v_{c}; and
- the automatic data processing apparatus (30) is adapted to determine freeness F as a function of the volume of suspension drained from the measuring chamber (10) by the time instant T1.

16. A measuring apparatus as claimed in claim 15, **characterized in that** to measure freeness F, the automatic data processing apparatus (30) is adapted to calculate the total volume Vₜₒₗₐₗ of liquid drained from the measuring chamber (10) from the time instant T0 to the time instant T1:
- the automatic data processing apparatus (30) is adapted to calculate the constant flow volume V_{cf} from the time instant T0 to the time instant T1 while the constant flow v_{c} is previously known;
- the automatic data processing apparatus (30) is adapted to calculate the difference result V by subtracting the constant flow volume V_{cf} and the previously known threshold volume Vₜₕ from the total volume Vₜₒₜₐₗ of liquid drained from the measuring chamber (10), and the automatic data processing apparatus is adapted to calculate freeness F of the suspension as a function of the difference result V.

17. A measuring apparatus as claimed in claim 15, **characterized in that** the measuring apparatus is adapted to measure the flow rate of the liquid draining from the measuring chamber (10) as a function of time until the flow substantially corresponds to the previously known flow rate v_{c} or is slower than v_{c}.

18. A measuring apparatus as claimed in claim 15, **characterized in that** the apparatus comprises a thermometer (36) to measure the temperature of the suspension and the thermometer (36) is adapted to feed measuring data to the automatic data processing apparatus (30) and the automatic data processing apparatus (30) is adapted to adjust the calculated freeness according to the temperature.

19. A measuring apparatus as claimed in claim 18, **characterized in that** the automatic data processing apparatus (30) is adapted to adjust the calculated freeness with respect to temperature according to the standard table.

20. A measuring apparatus as claimed in claim 15, **characterized in that** the measuring apparatus is adapted to measure the consistency of the suspension and the automatic data processing apparatus (30) is adapted to adjust the calculated freeness according to the consistency.

21. A measuring apparatus as claimed in claim 20, **characterized in that** the automatic data processing apparatus (30) is adapted to adjust the calculated freeness with respect to consistency according to the standard table.

22. A measuring apparatus as claimed in claim 15, **characterized in that** the measuring sensor (12) for measuring the drainage of the liquid from the measuring chamber (10) is adapted to measure acoustically the surface level of the suspension in the measuring chamber (10).

23. A measuring apparatus as claimed in claim 15, **characterized in that** the measuring sensor (12) for measuring the drainage of the liquid from the measuring chamber (10) is adapted to measure optically the surface level of the suspension in the measuring chamber (10).

24. A measuring apparatus as claimed in claim 16, **characterized in that** the previously known flow rate is V_{c} = 8.83 ml/s and the previously known threshold volume is Vₜₕ = 24.2 ml, and
the automatic data processing apparatus (30) is adapted to calculate freeness F corresponding to the CSF method so that freeness F substantially equals the difference result V or a corresponding value.

25. A measuring apparatus as claimed in claim 16, **characterized in that** the previously known flow rate is V_{c} = 1000 ml/(149 s ± 1 s) and the previously known threshold volume is Vₜₕ = 7.5 - 8.0 ml, and
the automatic data processing apparatus (30) is adapted to calculate freeness F corresponding to the SR method so that freeness F is substantially F = (1000 ml - V)/10 or a corresponding value.

26. A measuring apparatus as claimed in claim 15, **characterized in that** the measuring apparatus comprises an air valve (14) adapted to allow the suspension to flow through the wire (18) or the like after a predefined time at the time instant T0.

27. A measuring apparatus as claimed in claim 26, **characterized in that** the predefined time, after which the air valve (14) opens, is 5 s.

## Patentansprüche

1. Verfahren zum Messen einer Entwässerungsneigung, wobei in dem Verfahren:
- eine Messkammer (10) mit einem Sieb (18) oder dergleichen, welches an der Bodenfläche der Messkammer (10) angeordnet ist, mit einer zu messenden Suspension gefüllt wird,
- der Suspension zu einem Zeitpunkt T0 erlaubt wird, durch das Sieb (18) oder dergleichen zu strömen,
- nachdem die Strömung zu dem Zeitpunkt T0 startet, die Abnahme in der Suspension in der Messkammer (10) als eine Funktion der Zeit gemessen wird,
**dadurch gekennzeichnet, dass**
in dem Verfahren:
- ein Zeitpunkt T1 gesucht wird, bei welchem die Abnahme in der Suspension im Wesentlichen zu einer vorher bekannten Strömungsrate v_{c} korrespondiert; und
- eine Entwässerungsneigung F als eine Funktion des Volumens einer Suspension berechnet wird, die bei dem Zeitpunkt T1 von der Messkammer (10) abgeflossen ist.

2. Verfahren nach Anspruch 1,
**dadurch gekennzeichnet, dass**
zum Berechnen einer Entwässerungsneigung F als eine Funktion des Volumens einer Suspension, welches bis zu dem Zeitpunkt T1 abgeflossen ist:
- ein Gesamtvolumen V_{Total}, welches von der Messkammer (10) abgeflossen ist, von dem Zeitpunkt T0 bis zu dem Zeitpunkt T1 bestimmt wird;
- ein konstantes Strömungsvolumen V_{cf} von dem Zeitpunkt T0 bis zu dem Zeitpunkt T1 berechnet wird, während die konstante Strömung V_{c} vorher bekannt ist;
- ein Differenzergebnis V durch Subtrahieren des konstanten Strömungsvolumens V_{cf} und eines vorher bekannten Grenzvolumens Vₜₕ von dem Gesamtvolumen V_{Total} an Flüssigkeit berechnet wird, welches von der Messkammer (10) abgeflossen ist, und eine Entwässerungsneigung F der Suspension als eine Funktion des Differenzergebnisses V berechnet wird.

3. Verfahren nach Anspruch 1,
**dadurch gekennzeichnet, dass**
die Ausströmung der Suspension, welche von der Messkammer (10) abfließt, als eine Funktion der Zeit gemessen wird, bis die Strömung im Wesentlichen zu der vorher bekannten Strömung v_{c} korrespondierend oder langsamer als v_{c} ist.

4. Verfahren nach Anspruch 1,
**dadurch gekennzeichnet, dass**
die Temperatur der Suspension gemessen wird, und die berechnete Entwässerungsneigung F gemäß der Temperatur eingestellt wird.

5. Verfahren nach Anspruch 4,
**dadurch gekennzeichnet, dass**
die Temperatureinstellung gemäß einer Standardtabelle ausgeführt wird.

6. Verfahren nach Anspruch 1,
**dadurch gekennzeichnet, dass**
die Konsistenz der Suspension gemessen wird und die berechnete Entwässerungsneigung gemäß der Konsistenz eingestellt wird.

7. Verfahren nach Anspruch 6,
**dadurch gekennzeichnet, dass**
die Konsistenzeinstellung gemäß einer Standardtabelle ausgeführt wird.

8. Verfahren nach Anspruch 1,
**dadurch gekennzeichnet, dass**
der Abfluss der Flüssigkeit von der Messkammer (10) durch Messen des Flächenniveaus der Suspension in der Messkammer (10) akustisch gemessen wird.

9. Verfahren nach Anspruch 1,
**dadurch gekennzeichnet, dass**
der Abfluss der Flüssigkeit von der Messkammer (10) durch Messen des Flächenniveaus der Suspension in der Messkammer (10) optisch gemessen wird.

10. Verfahren nach Anspruch 1,
**dadurch gekennzeichnet, dass**
bei dem Verfahren alle Messungen und die Ergebnisberechnung mittels einer Mikroprozessorsteuerung automatisch ausgeführt werden.

11. Verfahren nach Anspruch 2,
**dadurch gekennzeichnet, dass**
die vorher bekannte Strömung v_{c} = 8,83 ml/s ist, und das vorher bekannte Grenzvolumen Vₜₕ = 24,2 ml ist, und eine Entwässerungsneigung F gemäß des CSF-Verfahrens im Wesentlichen F = V oder ein korrespondierender Wert ist.

12. Verfahren nach Anspruch 2,
**dadurch gekennzeichnet, dass**
die vorher bekannte Strömung v_{c} = 1000 ml/(149 s ± 1 s) ist, und das vorher bekannte Grenzvolumen Vₜₕ = 7,5 - 8,0 ml ist, und eine Entwässerungsneigung F gemäß des SR-Verfahrens im Wesentlichen F = (1000 ml - V)/10 oder ein korrespondierender Wert ist.

13. Verfahren nach Anspruch 1,
**dadurch gekennzeichnet, dass**
die zu messende Suspension für eine vordefinierte Zeit in der Messkammer (10) verbleibt, bevor sie durch das Sieb (18) oder dergleichen strömen kann.

14. Verfahren nach Anspruch 13,
**dadurch gekennzeichnet, dass**
die vordefinierte Zeit 5 s ist.

15. Messgerät zur Bestimmung einer Entwässerungsneigung, wobei das Messgerät eine Messkammer (10) mit einem oberen Deckel (34) und einem Bodenflächenverschluss (16) aufweist, welche die Messkammer (10), ein Luftventil (14) und ein Sieb (18) oder dergleichen dicht abdichten; wobei am Anfang des Messvorgangs die Messkammer (10) die zu messende Suspension beinhaltet; wobei der Verschluss (16) der Bodenfläche der Messkammer (10) gemacht ist, um geöffnet zu werden; wobei das Messgerät angeordnet ist, um der Suspension zu erlauben, zu einem Zeitpunkt T0 durch das Sieb (18) oder dergleichen zu strömen, wobei
- das Messgerät einen Messsensor (12) zum Messen des Abflusses der Flüssigkeit von der Messkammer (10) durch das Sieb (18) oder dergleichen als eine Funktion der Zeit aufweist; und
- das Messgerät ein automatisches Datenverarbeitungsgerät (30) aufweist, an dem der Sensor angeschlossen ist, um seine Messdaten einzugeben,
**dadurch gekennzeichnet, dass**
- das automatische Datenverarbeitungsgerät (30) eingerichtet ist, um nach einem Zeitpunkt T1 zu suchen, bei dem der Abfluss der Flüssigkeit aus der Messkammer (10) im Wesentlichen zu einer vorher bekannten Strömung v_{c} korrespondiert; und
- das automatische Datenverarbeitungsgerät (30) eingerichtet ist, um eine Entwässerungsneigung F als Funktion des Volumens der von der Messkammer (10) abfließenden Suspension bei dem Zeitpunkt T1 zu bestimmen.

16. Messgerät nach Anspruch 15,
**dadurch gekennzeichnet, dass**,
um eine Entwässerungsneigung F zu messen, das automatische Datenverarbeitungsgerät (30) eingerichtet ist, um das Gesamtvolumen V_{Total} der Flüssigkeit zu berechnen, welches von der Messkammer (10) von dem Zeitpunkt T0 bis zu dem Zeitpunkt T1 abgeflossen ist;
- das automatische Datenverarbeitungsgerät (30) eingerichtet ist, um das konstante Strömungsvolumen V_{cf} von dem Zeitpunkt T0 bis zu dem Zeitpunkt T1 zu berechnen, während die konstante Strömung v_{c} vorher bekannt ist;
- das automatische Datenverarbeitungsgerät (30) eingerichtet ist, um das Differenzergebnis V durch Subtrahieren des konstanten Strömungsvolumens V_{cf} und des vorher bekannten Grenzvolumens Vₜₕ von dem Gesamtvolumen V_{Total} an Flüssigkeit zu berechnen, welches von der Messkammer (10) abgeflossen ist, und wobei das automatische Datenverarbeitungsgerät eingerichtet ist, um eine Entwässerungsneigung F der Suspension als eine Funktion des Differenzergebnisses V zu berechnen.

17. Messgerät nach Anspruch 15,
**dadurch gekennzeichnet, dass**
das Messgerät eingerichtet ist, um die Strömungsrate der Flüssigkeit, welche von der Messkammer (10) abfließt, als eine Funktion der Zeit zu messen, bis die Strömung im Wesentlichen zu der vorher bekannten Strömung V_{c} korrespondierend oder langsamer als v_{c} ist.

18. Messgerät nach Anspruch 15,
**dadurch gekennzeichnet, dass**
das Gerät ein Thermometer (36) aufweist, um die Temperatur der Suspension zu messen, und das Thermometer (36) eingerichtet ist, um Messdaten zu dem automatischen Datenverarbeitungsgerät (30) einzugeben, und das automatische Datenverarbeitungsgerät (30) eingerichtet ist, um die berechnete Entwässerungsneigung gemäß der Temperatur einzustellen.

19. Messgerät nach Anspruch 18,
**dadurch gekennzeichnet, dass**
das automatische Datenverarbeitungsgerät (30) eingerichtet ist, um die berechnete Entwässerungsneigung mit Bezug auf eine Temperatur gemäß der Standardtabelle einzustellen.

20. Messgerät nach Anspruch 15,
**dadurch gekennzeichnet, dass**
das Messgerät eingerichtet ist, um die Konsistenz der Suspension zu messen, und das automatische Datenverarbeitungsgerät (30) eingerichtet ist, um die berechnete Entwässerungsneigung gemäß der Konsistenz einzustellen.

21. Messgerät nach Anspruch 20,
**dadurch gekennzeichnet, dass**
das automatische Datenverarbeitungsgerät (30) eingerichtet ist, um die berechnete Entwässerungsneigung mit Bezug auf eine Konsistenz gemäß der Standardtabelle einzustellen.

22. Messgerät nach Anspruch 15,
**dadurch gekennzeichnet, dass**
der Messsensor (12) zum Messen des Abflusses der Flüssigkeit von der Messkammer (10) eingerichtet ist, um das Flächenniveau der Suspension in der Messkammer (10) akustisch zu messen.

23. Messgerät nach Anspruch 15,
**dadurch gekennzeichnet, dass**
der Messsensor (12) zum Messen des Abflusses der Flüssigkeit von der Messkammer (10) eingerichtet ist, um das Flächenniveau der Suspension in der Messkammer (10) optisch zu messen.

24. Messgerät nach Anspruch 16,
**dadurch gekennzeichnet, dass**
die vorher bekannte Strömung v_{c} = 8,83 ml/s ist, und das vorher bekannte Grenzvolumen Vₜₕ = 24,2 ml ist, und
das automatische Datenverarbeitungsgerät (30) eingerichtet ist, um eine Entwässerungsneigung F, korrespondierend zu dem CSF-Verfahren zu berechnen, so dass eine Entwässerungsneigung F im Wesentlichen gleich dem Differenzergebnis V oder einem korrespondierenden Wert ist.

25. Messgerät nach Anspruch 16,
**dadurch gekennzeichnet, dass**
die vorher bekannte Strömung V_{c}= 1000 ml/(149 s ± 1 s) ist, und das vorher bekannte Grenzvolumen Vₜₕ = 7,5 - 8,0 ml ist, und das automatische Datenverarbeitungsgerät (30) eingerichtet ist, um eine Entwässerungsneigung F korrespondierend zu dem SR-Verfahren zu berechnen, so dass eine Entwässerungsneigung F im Wesentlichen F = (1000 ml - V)/10 oder ein korrespondierender Wert ist.

26. Messgerät nach Anspruch 15,
**dadurch gekennzeichnet, dass**
das Messgerät ein Luftventil (14) aufweist, welches eingerichtet ist, um der Suspension zu erlauben, nach einer vordefinierten Zeit zu dem Zeitpunkt T0 durch das Sieb (18) oder dergleichen zu strömen.

27. Messgerät nach Anspruch 26,
**dadurch gekennzeichnet, dass**
die vordefinierte Zeit, nach welcher das Luftventil (14) öffnet, 5 s ist.

## Revendications

1. Procédé de mesure de la vitesse de drainage, dans lequel :
- on remplit d'une suspension à mesurer une chambre de mesure (10) comprenant une toile métallique (18) ou un analogue déposée sur la surface de fond de la chambre de mesure (10),
- on laisse la suspension s'écouler à travers la toile métallique (18) ou analogue à un instant T0,
- après que l'écoulement a démarré à l'instant T0, on mesure la diminution de la suspension dans la chambre de mesure (10) en fonction du temps ;
**caractérisé en ce que**, dans le procédé,
- on recherche un instant T1 où la diminution de la suspension correspond essentiellement à un débit v_{c} connu antérieurement ; et
- on calcule la vitesse de drainage F en fonction du volume de suspension évacué de la chambre de mesure (10) à l'instant T1.

2. Procédé selon la revendication 1, **caractérisé en ce que**, pour calculer la vitesse de drainage F en fonction du volume de suspension évacué à l'instant T1 :
- on détermine un volume total Vₜₒₜₐₗ évacué de la chambre de mesure (10) depuis l'instant T0 jusqu'à l'instant T1 ;
- on calcule un volume d'écoulement constant V_{cf} depuis l'instant T0 jusqu'à l'instant T1, l'écoulement constant v_{c} étant connu antérieurement ;
- on calcule un résultat de différence V en soustrayant le volume à l'écoulement constant V_{cf} et un volume seuil Vₜₕ connu antérieurement du volume total Vₜₒₜₐₗ de liquide évacué de la chambre de mesure (10) et on calcule la vitesse de drainage F de la suspension en fonction du résultat des différences V.

3. Procédé selon la revendication 1, **caractérisé en ce qu'**on mesure l'échappement de la suspension évacuée de la chambre de mesure (10) en fonction du temps jusqu'à ce que l'écoulement corresponde essentiellement au débit v_{c} connu antérieurement ou qu'il soit plus lent que v_{c}.

4. Procédé selon la revendication 1, **caractérisé en ce qu'**on mesure la température de la suspension et on ajuste la vitesse de drainage calculée F en fonction de la température.

5. Procédé selon la revendication 4, **caractérisé en ce que** l'ajustement de la température est exécuté d'après une table d'étalonnage.

6. Procédé selon la revendication 1, **caractérisé en ce qu'**on mesure la consistance de la suspension et qu'on ajuste la vitesse de drainage calculée en fonction de la consistance.

7. Procédé selon la revendication 6, **caractérisé en ce qu'**on effectue l'ajustement de la consistance d'après une table d'étalonnage.

8. Procédé selon la revendication 1, **caractérisé en ce qu'**on mesure le drainage du liquide hors de la chambre de mesure (10) par voie acoustique, en mesurant le niveau de la surface de la suspension dans la chambre de mesure (10).

9. Procédé selon la revendication 1, **caractérisé en ce qu'**on mesure l'évacuation du liquide hors de la chambre de mesure (10) par voie optique, en mesurant le niveau de la surface de la suspension dans la chambre de mesure (10).

10. Procédé selon la revendication 1, **caractérisé en ce que**, dans le procédé, toutes les mesures et le calcul du résultat sont exécutés automatiquement à l'aide d'une commande par microprocesseur.

11. Procédé selon la revendication 2, **caractérisé en ce que** le débit connu antérieurement est v_{c} = 8,83 ml/s, le volume de seuil connu antérieurement est Vₜₕ = 24.2 ml et la vitesse de drainage F selon le procédé CSF est essentiellement F = V ou une valeur correspondante.

12. Procédé selon la revendication 2, **caractérisé en ce que** le débit connu antérieurement est v_{c} = 1000 ml/(149 s ± 1 s), le volume de seuil connu antérieurement est Vₜₕ = 7,5 - 8,0 ml et la vitesse de drainage F selon le procédé SR est essentiellement F = (1000 ml - V)/10 ou une valeur correspondante.

13. Procédé selon la revendication 1, **caractérisé en ce que** la suspension à mesurer reste dans la chambre de mesure (10) pendant un temps prédéfini avant qu'on ne la laisse s'écouler à travers la toile métallique (18) ou l'analogue.

14. Procédé selon la revendication 13, **caractérisé en ce que** le temps prédéfini est 5 s.

15. Dispositif de mesure destiné à mesurer la vitesse de drainage comprenant une chambre de mesure (10) qui comprend un couvercle supérieur (34) et un obturateur de surface de fond (16) qui ferme hermétiquement la chambre de mesure (10), une soupape à air (14) et une toile métallique (18) ou un analogue ; au début de l'opération de mesure, la chambre de mesure (10) contient la suspension à mesurer ; l'obturateur (16) de la surface de fond de la chambre de mesure (10) est amené à s'ouvrir ; le dispositif de mesure est agencé pour permettre à la suspension de s'écouler à travers la toile métallique (18) ou l'analogue à un instant T0, dans lequel
- le dispositif de mesure comprend un capteur de mesure (12) destiné à mesurer le drainage du liquide hors de la chambre de mesure (10) à travers la toile métallique (18) ou l'analogue en fonction du temps ;
- le dispositif de mesure comprend un dispositif de traitement automatique des données (30) auquel le capteur est amené à fournir ses données de mesure ;
**caractérisé en ce que** :
- le dispositif de traitement automatique des données (30) est adapté pour rechercher un instant T1 auquel le drainage du liquide hors de la chambre de mesure (18) correspond essentiellement à un débit connu antérieurement v_{c} ; et
- le dispositif de traitement automatique des données (30) est adapté pour déterminer la vitesse de drainage F en fonction du volume de suspension évacué de la chambre de mesure (10) à l'instant T1.

16. Dispositif de mesure selon la revendication 15, **caractérisé en ce que**, pour mesurer la vitesse de drainage F, le dispositif de traitement automatique des données (30) est adapté pour calculer le volume total Vₜₒₜₐₗ de liquide drainé de la chambre de mesure (10) depuis l'instant T0 jusqu'à l'instant T1 ;
- le dispositif de traitement automatique des données (30) est adapté pour calculer le volume d'écoulement constant V_{cf} depuis l'instant T0 jusqu'à l'instant T1, le débit constant V_{c} étant connu antérieurement ;
- le dispositif de traitement automatique des données (30) est adapté pour calculer le résultat de différence V en soustrayant le volume d'écoulement constant V_{cf} et le volume seuil Vₜₕ connu antérieurement du volume total Vₜₒₜₐₗ de liquide drainé de la chambre de mesure (10) et le dispositif de traitement automatique des données est adapté pour calculer la vitesse de drainage F de la suspension en fonction du résultat différence V.

17. Dispositif de mesure selon la revendication 15, **caractérisé en ce que** le dispositif de mesure est adapté pour mesurer le débit du liquide qui s'évacue de la chambre de mesure (10) en fonction du temps jusqu'à ce que le débit corresponde essentiellement au débit V_{c} connu antérieurement ou qu'il soit plus lent que v_{c}.

18. Dispositif de mesure selon la revendication 15, **caractérisé en ce que** le dispositif comprend un thermomètre (36) destiné à mesurer la température de la suspension et le thermomètre (36) est adapté pour transmettre les données de mesure au dispositif de traitement automatique des données (30) et le dispositif de traitement automatique des données (30) est adapté pour ajuster la vitesse de drainage calculée en fonction de la température.

19. Dispositif de mesure selon la revendication 18, **caractérisé en ce que** le dispositif de traitement automatique des données (30) est adapté pour ajuster la vitesse de drainage calculée par rapport à la température d'après la table d'étalonnage.

20. Dispositif de mesure selon la revendication 15, **caractérisé en ce que** le dispositif de mesure est adapté pour mesurer la consistance de la suspension et le dispositif de traitement automatique des données (30) est adapté pour ajuster la vitesse de drainage calculée en fonction de la consistance.

21. Dispositif de mesure selon la revendication 20, **caractérisé en ce que** le dispositif de traitement automatique des données (30) est adapté pour ajuster la vitesse de drainage calculée par rapport à la consistance d'après la table d'étalonnage.

22. Dispositif de mesure selon la revendication 15, **caractérisé en ce que** le capteur de mesure (12) destiné à mesurer le drainage du liquide hors de la chambre de mesure (10) est adapté pour mesurer le niveau de la surface de la suspension dans la chambre de mesure (10) par voie acoustique.

23. Dispositif de mesure selon la revendication 15, **caractérisé en ce que** le capteur de mesure (12) destiné à mesurer l'évacuation du liquide hors de la chambre de mesure (10) est adapté pour mesurer le niveau de la surface de la suspension dans la chambre de mesure (10) par voie optique.

24. Dispositif de mesure selon la revendication 16, **caractérisé en ce que** le débit connu antérieurement est v_{c} = 8,83 ml/s et le volume de seuil connu antérieurement est Vₜₕ = 24.2 ml, et
le dispositif de traitement automatique des données (30) est adapté pour calculer la vitesse de drainage F selon le procédé CSF de manière que la vitesse de drainage F soit essentiellement égale au résultat de différence V ou à une valeur correspondante.

25. Dispositif de mesure selon la revendication 16, **caractérisé en ce que** le débit connu antérieurement est V_{c} = 1000 ml/(149 s ± 1 s ) et le volume de seuil connu antérieurement est Vₜₕ = 7,5 - 8,0 ml, et
le dispositif de traitement automatique des données (30) est adapté pour calculer la vitesse de drainage F selon le procédé SR de telle manière que la vitesse de drainage F soit essentiellement F = (1000 ml - V)/10 ou une valeur correspondante.

26. Dispositif de mesure selon la revendication 15, **caractérisé en ce que** le dispositif de mesure comprend une soupape à air (14) adaptée pour laisser la suspension s'écouler à travers la toile métallique (18) ou l'analogue après un temps prédéterminé à l'instant T0.

27. Dispositif de mesure selon la revendication 26, **caractérisé en ce que** le temps prédéfini après lequel la soupape à air (14) s'ouvre est 5 s.
